(19) Europäisches Patentamt
European Patent Office
Office européen des brevets

(11) Publication number: **0 328 700 A1**

(12) **EUROPEAN PATENT APPLICATION**

(21) Application number: **88102251.1**

(22) Date of filing: **17.02.88**

(51) Int. Cl.4: **C07D 487/04 , A61K 31/505 , //(C07D487/04,239:00,231:00)**

(43) Date of publication of application: **23.08.89 Bulletin 89/34**

(84) Designated Contracting States: **AT BE CH DE ES FR GB IT LI NL SE**

(71) Applicant: **Yoshitomi Pharmaceutical Industries, Ltd.**
**35 Hiranomachi 3-chome Higashi-ku**
**Osaka-shi Osaka 541(JP)**

(72) Inventor: **Tsuda, Yoshinao**
**645.3 Higashisayamagaoka 1-chome**
**Tokorozawa-shi Saitama(JP)**
Inventor: **Mishina, Tadashi**
**2-102, 1-20 Koyodai 1-chome**
**Iruma-shi Saitama(JP)**
Inventor: **Obata, Minoru**
**4-36, Okidaimachi 1-chome**
**Nakatsu-shi Oita(JP)**
Inventor: **Araki, Kazuhiko**
**1212-4, Aza-Azema Oaza-Kamiikenaga**
**Nakatsu-shi Oita(JP)**
Inventor: **Inui, Jun**
**1129-78, Oaza-Noda**
**Iruma-shi Saitama(JP)**
Inventor: **Nakamura, Tadao**
**468-1, Shimomiyanaga 2-chome**
**Nakatsu-shi Oita(JP)**

(74) Representative: **von Kreisler, Alek, Dipl.-Chem. et al**
**Patentanwälte Von Kreisler-Selting-Werner**
**Deichmannhaus am Hauptbahnhof**
**D-5000 Köln 1(DE)**

(54) **4,7-Dihydropyrazolo(1,5-a)pyrimidine compound and pharmaceutical use thereof.**

(57) Isopropyl 5-(2-aminoethoxymethyl)-3-cyano-7-(2,3-difluorophenyl)-4,7-dihydropyrazolo[1,5-a]pyrimidine-6-carboxylate or pharmaceutically acceptable acid addition salt and/or hydrate thereof is disclosed.

Such compound has specifically mild and long lasting hypotensive activity and higher bioavailability by the oral administration.

EP 0 328 700 A1

## 4,7-Dihydropyrazolo[1,5-a]pyrimidine Compound and Pharmaceutical Use Thereof

This invention relates to isopropy 5-(2-aminoethoxymethyl)-3-cyano-7-(2,3-difluorophenyl)-4,7-dihydropyrazolo[1,5-a]pyrimidine-6-carboxylate, a pharmaceutically acceptable acid addition salt and/or a hydrate thereof and its pharmaceutical use.

EP-183848A discloses certain 4,7-dihydropyrazolo[1,5-a]pyrimidine-6-carboxylate compounds without any substituent on the pyrazole ring which are useful for the prophylaxis or treatment of circulatory diseases.

EP-217142A encompasses a staggering number of polyazaheterocyclic compounds, and discloses isopropyl 3-cyano-7-(2,3-difluorophenyl)-5-methyl-4,7-dihydropyrazolo[1,5-a]pyrimidine-6-carboxylate or ethyl 5-(2-aminoethoxymethyl)-7-(2-nitrophenyl)-4,7-dihydropyrazolo[1,5-a] pyrimidine-6-carboxylate which possess calcium antagonistic and/or agonistic activities.

As a result of the intensive investigations, the present inventors have found that the specific compound, i.e. isopropyl 5-(2-aminoethoxymethyl)-3-cyano-7-(2,3-difluorophenyl)-4,7-dihydropyrazolo[1,5-a] pyrimidine-6-carboxylate [hereinafter referred to as compound (I)] inclusive of salt and/or hydrate form thereof exhibits more preferable and specific pharmacological activities than the above-mentioned prior art compounds. The compound (I) of the present invention shows strong calcium antagonistic activity and, specifically, mild and long lasting hypotensive activity, high selective coronary vasodilating activity, and higher bioavailability by the oral administration. Further, the compound (I) of the present invention exhibits potent diuretic activity and renal blood flow increasing activity, but weak heart-depressive activity.

Therefore, the present invention relates to isopropyl 5-(2-aminoethoxymethyl)-3-cyano-7-(2,3-difluorophenyl)-4,7-dihydropyrazolo[1,5-a]pyrimidine-6-carboxylate, a pharmaceutically acceptable acid addition salt and/or a hydrate thereof.

The present invention also relates to a pharmaceutical composition comprising a therapeutically effective amount of the compound (I) or a pharmaceutically acceptable acid addition salt and/or hydrate thereof with a pharmaceutically acceptable additives.

Further, the present invention relates to a use of the compound (I) or a pharmaceutically acceptable acid addition salt and/or hydrate thereof for the manufacture of a drug for the improvement or treatment of hypertention, angina pectoris, heart failure or disorder in cerebral circulation.

According to the present invention, the compound (I) can be prepared by reacting a compound of the formula:

$$H_2NCH_2CH_2OCH_2\overset{O}{\overset{\|}{C}}\,CH_2\,\overset{O}{\overset{\|}{C}}\,OCH(CH_3)_2 \qquad (II)$$

or its N-protected form with 2,3-difluorobenzaldehyde and reacting the resultant compound with 3-amino-4-cyanopyrazole, and then deblocking the N-protected group to form a free amino group.

The reaction of the compound (II) or its N-protected form with 2,3-difluorobenzaldehyde is usually carried out by refluxing with removing water formed at room temperature or under heating in the presence of a conventional acid catalyst, base catalyst or both in an inert solvent.

The N-protected form of the compound of formula (II) includes, for example, N-butoxycarbonyl-, N-diazo-, N-benzyloxycarbonyl-, N-trichloroethoxycarbonyl-, N-formyl-, N-trifluoroacetyl-, N-phthaloyl- or N-benzylidene- compound, and the preferable N-protected compound of formula (II) is isopropyl 4-[2-(phthalimido)ethoxy]-3-oxobutylate.

The second reaction of the resultant compound which is a compound of the formula:

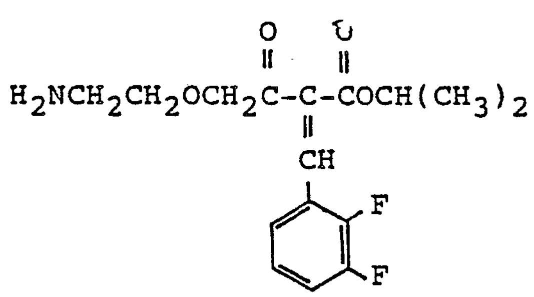

(III)

or its N-protected form with 3-amino-4-cyanopyrazole is usually carried out by heating at a temperature up

to the boiling point of the solvent employed for several hours to several scores of hours in an inert solvent.

The deblocking reaction of the N-protected group is usually carried out by using a coventional acid or base, hydrazine hydrate or zinc and the like or by subjecting to hydrogenation, preferably under heating for several hours to several scores of hours. The N-protected group can be deblocked as follows: debutoxycarbonylation with a conventional acid; removal of azido group by hydrogenation; debenzyloxycarbonylation with a conventional acid or hydrazine hydrate, or by hydrogenation; detrichloroethoxycarbonylation with a conventional acid or zinc; deformylation with a conventional acid or base, or hydrazine hydrate or by hydrogenation; detrifluoroacetylation with a conventional acid or base; removal of benzylidene group with a conventional acid; and dephthaloylation with a conventional base or hydrazine hydrate.

The inert solvent employed in the above reaction includes, for example, a lower alkanol (e.g. methanol, ethanol, propanol, isopropanol, butanol, tert-butanol or ethylene glycol), an aromatic hydrocarbon (e.g. benzene, toluene or xylene), an ether (e.g. diethyl ether, diisopropyl ether, dimethoxyethane, diethylene glycol dimethyl ether, tetrahydrofuran or dioxane), a halogenated lower alkane (e.g. dichloromethane, dichloroethan, chloroform or tetrachloromethane), an acid amide (e.g. dimethylformamide, N-methylpyrrolidone or hexamethylphosphorotriamide), a lower alkanecarboxylic acid or an ester thereof (e.g. formic acid, acetic acid, propionic acid, methyl acetate, ethyl acetate, butyl acetate or methyl propionate), a nitrated lower alkane (e.g. nitromethane or nitroethane), dimethylsulfoxide, sulforane, water or a mixture thereof.

The compound of formula (I) having an asymmetric carbon atom can be prepared as a racemate or an optically active isomer. The racemate can be devided into a desired optical isomer by means of, for example, a fractional recrystallization of a salt with an optically active base or acid; passing through the column filled with an optically active stationary phase; producing an amide bond with an optically active carboxylic acid, and then separating the formed diastereomer by using a fractional recrystallization or column chromatography. Preferably, the optically active isomers of the compound (I) can be obtained by deviding the N-protected racemate into the individual optical isomers by means of the above-mentioned method, and then deblocking the N-protected group of the N-protected optical isomer.

The compound (I), optical isomers thereof can, if desired, be converted into acid addition salts thereof with an inorganic acid (e.g. hydrochloric acid, hydrobromic acid, sulfuric acid or phosphoric acid), an organic acid (e.g. acetic acid, propionic acid, glycollic acid, succinic acid, maleic acid, fumaric acid, malic acid, tartaric acid, oxalic acid, citric acid, benzoic acid, cinnamic acid, mandelic acid, salicylic acid, 2-acetoxybenzoic acid, nicotinic acid or isonicotinic acid), an organic sulfonic acid (e.g. methanesulfonic acid, ethanesulfonic acid, 2-hydroxyethanesulfonic acid, benzenesulfonic acid, p-toluenesulfonic acid or naphthalene-2-sulfonic acid), ascorbic acid, lysine or glutamic acid. Among them, pharmaceutically acceptable acid addition salt form is desirable for the purpose of the present invention. The compound (I) or acid addition salts can also exist as a hydrate form (e.g. monohydrate, dihydrate, hemihydrate, sesquihydrate or 1/4 hydrate).

The compound (I), preferably as the hydrochloride of the present invention shows strong calcium antagonistic activity, and mild and long lasting hypotensive activity in a pharmacological experiments using spontaneous hypertensive rats, anesthetized or non-anesthetized dogs or rabbits. The effect of the compound (I) of the present invention on the coronary blood flow is highly selective, and additionally the compound (I) exhibits potent improvement of renal function (e.g. $Na^+$ diuretic activity) and renal vasodilating activity, but weak heart-depressive activity. Furthermore, the bioavailability by the oral administration is much higher and the safety margin is broader. The compound (I) has a better solubility in water and good stability on exposure to light.

Accordingly, the compound (I) or a pharmaceutically acceptable acid addition salt and/or hydrate thereof can be characteristically administered by the oral route in a form of tablets, sugar coated tablets, film coated tablets, granules, powders or capsules with high safety to the patients suffering from hypertention, angina pectoris, disorder of cerebral circulation and the like.

The compounds of the present invention, when used as drugs, can be administered in the form of, preferably,powder, tablets, sugar coated or film coated tablets, capsules or granules and injectable solutions, suppositories, ointments or the like by mixing a therapeutically effective amount of the compound (I) or salt and/or hydrate thereof with a pharmaceutically acceptable additives such as carrier, excipient, diluent or the like. The dose for human adults usually ranges from 5 mg to 50 mg, but it may vary depending upon the age, body weight, and severity of the conditions to be treated.

| Formulation Example 1 : Tablets | |
|---|---|
| | 10 mg tablets |
| Compounds (I)<br>Lactose<br>Corn starch<br>Crystalline cellulose<br>Methyl cellulose<br>Talc<br>Magnesium stearate | 10.0 mg<br>64.5<br>20.0<br>10.0<br>1.0<br>4.0<br>0.5<br>110.0 mg |

| Formulation Example 2 : 5 % Powder | |
|---|---|
| Compounds (I)<br>Lactose<br>Corn starch<br>Talc | 5.0 %<br>64.5<br>30.0<br>0.5<br>100.0 % |

Example 1

To a solution of 3.0 g of isopropyl 4-[2-(phthalimido)ethoxy]-3-oxobutylate and 1.5 g of 2,3-difluorobenzaldehyde in 30 ml of benzene is added two drops of acetic acid and piperidine respectively and the mixture is refluxed for 3 hours with Dean-Stark trap. The solvent is distilled off and to the residue are added 1.1 g of 3-amino-4-cyanopyrazole and 10 ml of dimethylformamide, and then heated at 100° C for 6 hours. After the solvent is distilled off, the residue is purified by column chromatography on silica gel and crystallized from ethanol to give 1.65 g of isopropyl 3-cyano-7-(2,3-difluorophenyl)-5-[2-(phthalimido)-ethoxymethyl]-4,7-dihydropyrazolo[1,5-a]pyrimidine-6-carboxylate, melting at 176-177° C.

To a solution of 1.3 g of isopropyl 3-cyano-7-(2,3-difluorophenyl)-5-[2-(phthalimido)ethoxymethyl]-4,7-dihydropyrazolo[1,5-a]pyrimidine-6-carboxylate in a mixed solvent of 12 ml of methanol and 12 ml of chloroform is added 142 $\mu$l of hydrazine hydrate and the mixture is refluxed at 50° C for 5 hours. After the solvent is distilled off, to the residue is added aqueous sodium hydrogencarbonate and then extracted with ethyl acetate. The organic layer is washed with water, dried over sodium sulfate and ethanolic hydrochloric acid is added to convert into hydrochloride. The obtained crystals are recrystallized from methanol to give 0.4 g of isopropyl 5-(2-aminoethoxymethyl)-3-cyano-7-(2,3-difluorophenyl)-4,7-dihydropyrazolo [1,5-a]-pyrimidine-6-carboxylate hydrochloride 1/4 hydrate.
Melting point : 231-233° C with decomposition
Mass spectrum : m/z 417
Thin layer chromatography : Rf=0.26 (Merck ART 5715, chloroform : methanol = 4 : 1)

The foregoing isopropyl 3-cyano-7-(2,3-difluorophenyl)-5-[2-(phthalimido)ethoxymethyl]-4,7-dihydropyrazolo[1,5-a]-pyrimidine-6-carboxylate is subjected to an optically active column (YMC-Pack AK-43) and eluted with a mixture of n-hexane-ethyl acetate-ethanol (55 : 45 : 1) to purify and separate it to give two optical isomers. Each optical isomer is treated in a similar manner as mentioned above to give d-form [melting at 217-218° C with decomposition, $[\alpha]_D^{20}$ = +68° (c=0.25, ethanol)] and -form [melting at 217-218° C with decomposition, $[\alpha]_D^{20}$ = -70° (c=0.25, ethanol)] of isopropyl 5-(2-aminoethoxymethyl)-3-cyano-7-(2,3-difluorophenyl)-4,7-dihydropyrazolo[1,5-a]pyrimidine-6-carboxylate hydrochloride, respectively.

Isopropyl 4-[2-(phthalimido)ethoxy]-3-oxobutylate, as the starting compound, is prepared as follows:
To a suspension of 6 g of 2-(phthalimido)ethoxyacetic acid in dichloromethane are added 2.05 ml of thionyl chloride and 3 drops of dimethylformamide, and the mixture is stirred for 2 days. The solvent is distilled off to give 2-(phthalimido)ethoxyacetyl chloride. A solution of 2-(phthalimido)ethoxyacetyl chloride in 10 ml of dichloromethane is added dropwise to a solution, maintained at -5° C, of 3.6 g of Meldrum's acid in 20 ml of

dichloromethane and 4.3 ml of pyridine. Then, the mixture is stirred overnight at room temperature. To the resultant mixture is added 2 normal hydrochloric acid and the dichloromethane layer is extractred. The extract is washed with sodium chloride solution three times, dried over sodium sulfate and the solvent is distilled off. To the residue is added 50 ml of isopropyl alcohol and refluxed for 5 hours. The solvent is distilled off and the residue is purified by column chromatography on silica gel to give 3.7 g of isopropyl 4-[2-(phthalimido)ethoxy]-3-oxobutylate.

## Claims

1. Isopropyl 5-(2-aminoethoxymethyl)-3-cyano-7-(2,3-difluorophenyl)-4,7-dihydropyrazolo[1,5-a]-pyrimidine-6-carboxylate or a pharmaceutically acceptable acid addition salt and/or hydrate thereof.

2. A pharmaceutical composition comprising a therapeutically effective amount of isopropyl 5-(2-aminoethoxymethyl)-3-cyano-7-(2,3-difluorophenyl)-4,7-dihydropyrazolo[1,5-a]pyrimidine-6-carboxylate or a pharmaceutically acceptable acid addition salt and/or hydrate thereof with a pharmaceutically acceptable additive.

3. A use of isopropyl 5-(2-aminoethoxymethyl)-3-cyano-7-(2,3-difluorophenyl)-4,7-dihydropyrazolo[1,5-a]pyrimidine-6-carboxylate or a pharmaceutically acceptable acid addition salt and/or hydrate thereof for the manufacture of a drug for the improvement or treatment of hypertention, angina pectoris, heart failure or disorder in cerebral circulation.

European Patent Office

# EUROPEAN SEARCH REPORT

Application Number

EP 88 10 2251

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 217 142 (YOSHITOMI)<br>* Claims 1,8; page 21; examples 11,162 *<br>----- | 1,2 | C 07 D 487/04<br>A 61 K 31/505//<br>(C 07 D 487/04<br>C 07 D 239:00<br>C 07 D 231:00 ) |
| | | | TECHNICAL FIELDS SEARCHED (Int. Cl.4) |
| | | | C 07 D 487/00<br>A 61 K 31/00 |

The present search report has been drawn up for all claims

| Place of search | Date of completion of the search | Examiner |
|---|---|---|
| THE HAGUE | 05-10-1988 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document